# EUROPEAN PATENT APPLICATION

(11) **EP 4 691 433 A1**
(43) Date of publication of application: **11.02.2026**
(21) Application number: 24192951.2
(22) Date of filing: 05.08.2024
(51) Int. Cl.: A61F 7/00, A61H 33/00, A47K 3/02

(54) **ICE BATH WITH A MAIN BASIN AND AN EQUALIZING BASIN**

(71) Applicant: Icetubs B.V., 1055 RX Amsterdam (NL)
(72) Inventor: Kraai, Chiel-Jan, 1056 XN Amsterdam (NL); Diels, Laurens, 1052 EG Amsterdam (NL)
(74) Representative: Keilitz, Wolfgang

(57) **Abstract**

The invention relates to an ice bath (1) with the features of claim 1.

## Description

The invention relates to an ice bath with a main basin and an equalizing basin.

An ice bath is usually a small pool filled with water at a temperature of around three degrees Celsius that is used for cold therapy. Ice baths help to relieve pain, soothe sore muscles and joint pain and, according to studies, even make you slim, as white fat is converted to brown fat and burned off. Regular ice bathing also strengthens the immune system and has a revitalizing effect on the circulation.

In public wellness areas where cold baths are used in quick succession by different people, it is important to comply with hygiene requirements. Well-known ice baths include built-in filters, strainers or skimmers for this purpose, which ensure consistently high water quality. The water in the ice bath is also replaced regularly, which, however, increases water consumption and energy consumption.

It is therefore an object of the present invention to further improve the water retention system of ice baths and to minimize water consumption.

This object is solved in accordance with the invention by the features specified in claim 1. Further embodiments of the invention are shown in the sub-claims.

According to the invention, an ice bath is proposed comprising a water inlet and a water outlet, a main basin and an equalizing basin which are separated from each other by an intermediate wall, an overflow via which water can flow from the main basin into the equalizing basin, a cooling device which draws water from the equalizing basin, cools it and directs it into the main basin, from where it can flow back into the equalizing basin via the overflow. The ice bath according to the invention further comprises a valve, in particular a float valve or ball valve, which regulates the filling of the equalizing basin, the valve being configured so that it interrupts feeding of fresh water when the water level in the equalizing basin has reached a predetermined level which is lower than the level in the main basin, in particular a maximum level. The proposed ice bath has the advantage that a large proportion of the water displaced by a person can be collected in the equalizing basin and reused, thereby reducing both water and energy consumption.

According to a preferred embodiment of the invention, the valve is realized as a float valve or ball valve. The valve is preferably arranged in the equalizing basin.

In one embodiment, the main basin has a main water outlet or drain, respectively, into the waste water network and the equalizing basin has a second water outlet or drain into the main basin, so that the water from the equalizing basin first drains into the main basin and then further through the main water outlet into the waste water network.

A drain valve can be provided at the second water outlet of the equalizing basin, by means of which the equalizing basin can be drained. The drain valve is preferably an automatically opening valve and in particular a one-way valve, which is closed as long as the main basin still contains water and which opens automatically as soon as the back pressure from the main basin falls below a certain threshold.

In a special embodiment of the invention, the equalization basin has an overflow via which water can flow into the public sewage network if a maximum level in the equalization basin is exceeded.

One embodiment of the ice bath comprises one or more filters arranged in the equalizing basin, through which the water is sucked in by the cooling device during operation and pumped in a circle.

The ice bath preferably also includes a shut-off valve located in the main water drain, by means of which the water in the main basin can be drained into the waste water network.

The cooling device is preferably switched off as long as the equalizing basin is filled with water. It preferably switches on automatically when the level in the equalizing basin has risen to such an extent that the valve interrupts the water supply. For the purpose of controlling the cooling device, the valve can, for example, close an electrical circuit of the cooling device.

The ice bath according to the invention is preferably designed such that the water in the main basin flows over an upper edge of the intermediate wall during normal operation and flows from the main basin into the equalizing basin and from there is pumped back into the main basin by the cooling device.

In a preferred embodiment, the equalizing basin is dimensioned so that it can hold at least 50 I, in particular at least 80 I, of additional water from the main basin without water running off via the overflow of the equalizing basin.

### Brief description of the drawings

The invention is explained in more detail below with reference to the accompanying drawings. In the drawings:
Fig. 1 is a perspective view of an ice bath according to an embodiment of the invention;
Fig. 2 is a sectional view through the ice bath of Fig. 1, in which the equalizing basin can be seen in more detail;
Fig. 3 is a view of some components of the ice bath of Fig. 1 and
Fig. 4 is a hydraulic circuit diagram of the ice bath of Fig. 1.

### Embodiments of the invention

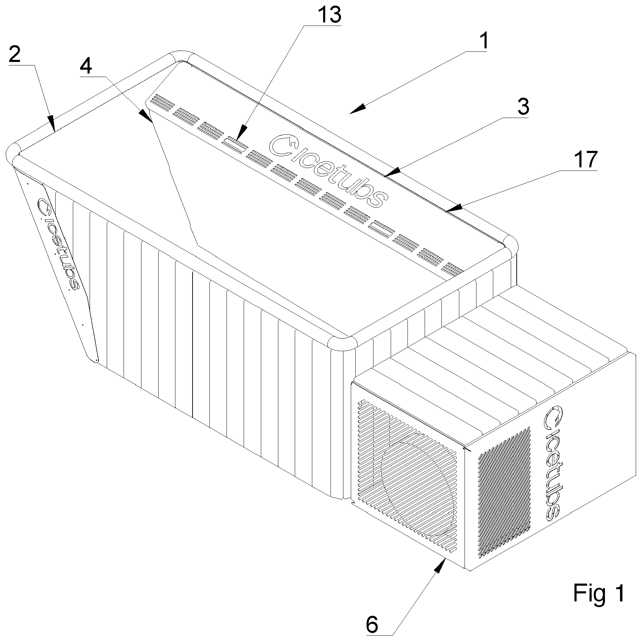

Fig. 1 shows a perspective view of an ice bath 1 according to one embodiment of the invention. The ice bath 1 comprises a main basin 2, which is designed for one or two persons, and an equalizing basin 3 arranged next to it. The two basins 2, 3 are separated from each other by a partition wall 4. In normal operation, the main basin 2 is filled with water up to the upper edge of the partition wall 4. The water level in the equalizing basin 3 is lower than the level in the main basin 2.

The ice bath 1 also includes a cooling device 6, which draws water from the equalizing basin 3, cools it and pumps it into the main basin 2. The upper edge of the intermediate wall 4 forms an overflow 13, via which the water from the main basin 2 flows back into the equalizing basin 3. The equalizing basin 3 is covered with a panel 17 with slotted openings through which the water flows in.

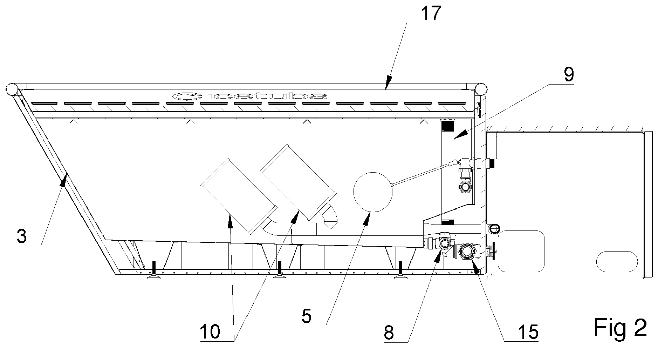

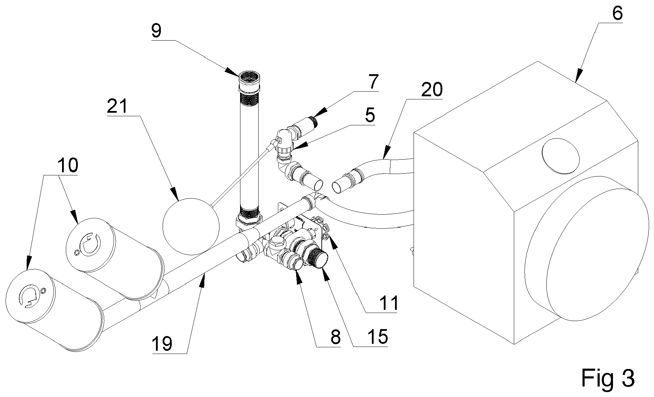

There is also a valve 5 in the equalizing basin 3 (see Fig. 2), which regulates the filling of the equalizing basin 3 with fresh water from the public water network 7. The valve 5 is designed so that fresh water flows in as long as the fill level in the equalizing basin 3 is lower than a predetermined level. Filling is stopped when the water level in the equalizing basin 3 has reached the specified level. In the embodiment shown, the valve 5 is designed as a float valve 14 having a float 21 (see Fig. 3).

During operation of the ice bath 1, the water is constantly pumped in a circle by the cooling device 6 and cooled, if necessary. As soon as a person enters the bath, the water displaced by the person runs over the intermediate wall 4 into the equalizing basin 3. The equalizing basin 3 has an overflow 9 via which water can flow into the public waste water network 18 if a maximum level in the equalizing basin 3 is exceeded. It is preferably dimensioned so that it can hold at least 50 liters, in particular at least 80 liters, of additional water from the main basin without water flowing out via the overflow 9.

Fig. 2 shows a sectional view of the ice bath 1 of Fig. 1, which provides a view of the equalizing basin 3. As can be seen, several filters 10 and the float valve 5 described above are arranged in the equalizing basin 3.

Fig. 3 shows further components of the ice bath 1 of Fig. 1, namely the cooling device 6, the filters 10 and a pipe 19 through which water is drawn from the equalizing basin 3. After the water has passed through the cooling device 6, the cooled water is pumped into the main basin 2 via a pipe 20. Fig. 3 also shows: a main water drain 15 with a shut-off valve 11 and a second water drain 8, via which water can flow out of the equalizing basin 3 into the main basin 2.

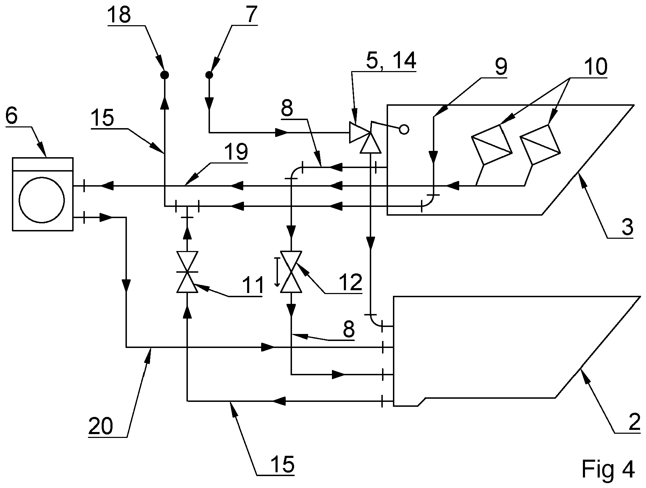

The structure of the ice bath 1 with its individual components and their function are explained in more detail below using the hydraulic circuit diagram in Fig. 4.

Fig. 4 shows a schematic representation of the ice bath 1 with the main basin 2 and the equalizing basin 3 described above. The ice bath 1 also comprises the cooling device 6, which draws water from the equalizing basin 3 via the filters 10, cools the water and then pumps it into the main basin 2.

The main basin 2 further has a main water outlet 15 into the waste water network 18. The equalizing basin 3 has a second water outlet 8 into the main basin 2, via which the water from the equalizing basin 3 first flows into the main basin 2 and then further through the main water outlet 15 into the waste water network 18. The second water outlet of the equalizing basin 3 comprises a drain valve 12, which is preferably an automatically opening valve and in particular a one-way valve, which is closed as long as the main basin 2 still contains water and which opens automatically as soon as the back pressure from the main basin 2 falls below a certain threshold.

The ice bath 1 according to Fig. 4 also comprises a shut-off valve 11 arranged in the main water drain 15, by means of which the water in the main basin 2 can be drained into the waste water network 18.

The cooling device 6 is preferably switched off as long as fresh water is fed into the equalizing basin 3 from the public water supply. It preferably switches on automatically when the level in the equalizing basin 3 has risen to such an extent that valve 5 interrupts the water supply. For this purpose, the valve 5 can, for example, close an electrical circuit of the cooling device 6.

## Claims

1. An ice bath (1) having:
- a water inlet (7) and a water outlet (15),
- a main basin (2) and an equalizing basin (3), which are separated from each other by an intermediate wall (4),
- an overflow (13) via which water can flow from the main basin (2) into the equalizing basin (3),
- a cooling device (6) which takes water from the equalizing basin (3), cools it and feeds it into the main basin (2), from where it can flow back into the equalizing basin (3) via the overflow (13),
- a valve (5) which regulates the filling of the equalizing basin (3) with water from the water inlet (7), the valve (5) being configured so that it interrupts the water feed when the water level in the equalizing basin (3) has reached a predetermined level which is lower than the level in the main basin (2).

2. The ice bath (1) according to claim 1, **characterized in that** the valve (5) is a float valve (14).

3. The ice bath (1) according to claim 1 or 2, **characterized in that** the valve (5) is arranged in the equalizing basin (3).

4. The ice bath (1) according to one of the preceding claims, **characterized in that** the main basin (2) has a main water outlet (15) into the waste water network (18) and the equalizing basin (3) has a second water outlet (8) into the main basin (2), so that the water from the equalizing basin (3) flows first into the main basin (2) and then further through the main water outlet (15) into the waste water network (18).

5. The ice bath (1) according to claim 4, **characterized in that** the second water outlet (8) of the equalizing basin (3) has a valve (12), in particular an automatically opening valve and in particular a one-way valve.

6. The ice bath (1) according to one of the preceding claims, **characterized in that** the equalizing basin (3) has an overflow (9) via which water can flow into the public waste water network (18) if a maximum level is exceeded.

7. The ice bath (1) according to one of the preceding claims, **characterized in that** one or more filters (10) are arranged in the equalizing basin (3), through which the water flows to the cooling device (6).

8. The ice bath (1) according to one of the preceding claims, **characterized in that** the main water drain (15) comprises a shut-off valve (11) by means of which the water in the main basin (2) can be drained into the waste water network (18).

9. The ice bath (1) according to one of the preceding claims, **characterized in that** the cooling device (6) is switched on when the water level in the equalizing basin (3) has reached the predetermined level at which the valve (5) interrupts the water feed into the equalizing basin (3).

10. The ice bath (1) according to one of the preceding claims, **characterized in that** it is designed such that the water in the main basin (2) flows over an upper edge of the intermediate wall (4) during normal operation and flows from the main basin (2) into the equalizing basin (3) and from there slides through the cooling device (6) and is pumped back into the main basin (2).

11. The ice bath (1) according to one of the preceding claims, **characterized in that** the equalizing basin (3) is dimensioned such that it can hold at least 50 I, in particular at least 80 I, of additional water from the main basin (2) without water running off via an overflow (9).
